(19) Europäisches Patentamt
European Patent Office
Office européen des brevets

(11) **EP 2 517 618 A2**

(12) **EUROPEAN PATENT APPLICATION**

(43) Date of publication:
**31.10.2012 Bulletin 2012/44**

(51) Int Cl.:
*A61B 3/15* (2006.01)          *A61B 3/10* (2006.01)
*A61B 3/12* (2006.01)

(21) Application number: **12165393.5**

(22) Date of filing: **24.04.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.04.2011 JP 2011100132**
**19.01.2012 JP 2012009428**

(71) Applicant: **CANON KABUSHIKI KAISHA**
**Ohta-ku**
**Tokyo 146-8501 (JP)**

(72) Inventors:
• **Saito, Kenichi**
  **Ohta-ku, Tokyo (JP)**
• **Sugita, Mitsuro**
  **Ohta-ku, Tokyo (JP)**

(74) Representative: **Williamson, Brian**
**Canon Europe Ltd**
**European Patent Department**
**3 The Square**
**Stockley Park**
**Uxbridge, Middlesex UB11 1ET (GB)**

(54) **Ophthalmic apparatus, method of controlling ophthalmic apparatus and storage medium**

(57) An ophthalmic apparatus comprises: a first optical system (42, 43) which irradiates the same region on a retina with a plurality of illumination light beams through separate positions on a pupil of an eye to be examined; and image generation means (31) for generating a two-dimensional image of the retina based on return light from the eye.

# F I G.  1

EP 2 517 618 A2

**Description**

BACKGROUND OF THE INVENTION

Field of the Invention

The present invention relates to an ophthalmic apparatus, a method of controlling the ophthalmic apparatus, and a storage medium and, more particularly, to an ophthalmic apparatus configured to acquire a two-dimensional image of the retina of an eye, such as an ophthalmoscope using a laser beam as illumination light, a method of controlling the ophthalmic apparatus, and a storage medium.

Description of the Related Art

As fundus imaging apparatuses which observe and capture a two-dimensional front image of the retina of an eye to be examined, a fundus camera, scanning laser ophthalmoscope (SLO), and the like are well known. These apparatuses are designed to acquire a retinal image by illuminating the retina as a target to be imaged with illumination light and forming reflected/backscattered light from the retina into an image on an imaging element. As this illumination light, light having a near-infrared wavelength is often used, which is not much absorbed or scattered by the living tissue (including the anterior ocular segment and corpus vitreum) in the eye to be examined.

As an illumination scheme, there is available a scheme of area-illuminating an imaging target region of the retina and acquiring an image by forming the image on a two-dimensional imaging element. There is also available a scheme of forming a two-dimensional image by illumination with illumination light in a small spot or linear form, two-dimensionally or one-dimensionally scanning the illumination light, and capturing reflected/backscattered light of the illumination light.

For example, WO06/046627 discloses a technique of acquiring a front retinal image by focusing two illumination light beams once at portions near the anterior ocular segment, illuminating different regions on the retina with light spread from the portions, and forming reflected light into an image on an imaging element having a two-dimensional matrix of pixels.

U.S. Patent No. 6,758,564 discloses the arrangement of an SLO configured to form illumination light into a linear image on the retina, one-dimensionally perform scanning in a direction perpendicular to the linear direction of the linear illumination light, and receive the light reflected by the retina by a one-dimensional line CCD array detector corresponding to the linear direction of the light beam.

Japanese Patent Laid-Open No. 2005-531346 has proposed an arrangement combining an SLO optical system designed to two-dimensionally scan a point-like small spot on the retina with an OCT (Optical Coherence Tomography) which can noninvasively obtain a tomogram of the eye to be examined. This arrangement allows to simultaneously acquire a retinal tomogram and a front image of the fundus.

In the arrangements disclosed in WO06/046627 and U.S. Patent No. 6,758,564, when uniformly spreading light in a two-dimensional or one-dimensional direction, assuming that an examination region of about 8 to 12 mm is required on the retina, it is necessary to focus illumination light to several $\mu$m on the anterior ocular segment. In addition, it is difficult to acquire a bright, high-contrast image unless energy per unit area on the retina is ensured for illumination light. However, increasing the amount of illumination light will increase the energy per area on the anterior ocular segment such as the cornea or crystalline lens. This may strain the anterior ocular segment. For this reason, some limitation is imposed on the amount of illumination light. This therefore limits the brightness of an acquired image.

According to Japanese Patent Laid-Open No. 2005-531346, since the SLO and the OCT are generally configured to set, for the respective illumination light beams, pivot points for beam scanning at the center of the pupil of the eye to be examined, the two beams are always superimposed and applied at the same position on the anterior ocular segment (pupil). For this reason, to simultaneously obtain SLO and OCT images, it is necessary to limit the amounts of the respective illumination beams. This will limit the brightness levels of images obtained by the two schemes.

In any case, if the anterior ocular segment at an incident beam position is cloudy due to a disease like cataract, the amount of illumination light reaching the retina decreases, resulting in a deterioration in image quality.

SUMMARY OF THE INVENTION

In consideration of the above problems, the present invention provides a technique of acquiring a bright, high-contrast retinal image upon reducing the load on an object to be examined.

According to one aspect of the present invention, there is provided an ophthalmic apparatus comprising as specified in claim 1.

According to one aspect of the present invention, there is provided a method of controlling an ophthalmic apparatus as specified in claim 19.

Further features of the present invention will be apparent from the following description of exemplary embodiments with reference to the attached drawings.

BRIEF DESCRIPTION OF THE DRAWINGS

Fig. 1 is a view showing the arrangement of an ophthalmic apparatus according to the first embodiment;

Fig. 2 is a sectional view of an incident beam on the eye to be examined in the first embodiment;

Fig. 3 is a sectional view of return light from the retina in the first embodiment;

Fig. 4 is a view showing the incident positions of two illumination light beams at the time of observation of the anterior ocular segment of the eye to be examined from a visual axis direction in the first embodiment;

Fig. 5 is a view showing the arrangement of an ophthalmic apparatus according to the second embodiment;

Fig. 6 is a view showing the incident positions of two illumination light beams at the time of observation of the anterior ocular segment of the eye to be examined from the visual axis direction in the second embodiment;

Fig. 7 is a view showing the positional shift between beams on the retina in the second embodiment;

Fig. 8 is a view showing the arrangement of an ophthalmic apparatus according to the third embodiment;

Fig. 9 is a view showing the incident positions of three illumination light beams at the time of observation of the anterior ocular segment of the eye to be examined from the visual axis direction in the third embodiment;

Fig. 10 is a sectional view of an incident beam on the eye to be examined in the third embodiment;

Fig. 11 is a view showing the incident positions of three illumination light beams at the time of observation of the anterior ocular segment of the eye to be examined from the visual axis direction in the third embodiment;

Fig. 12 is a view showing the propagation of reflected ghost light from a lens surface in the fourth embodiment; and

Fig. 13 is a view showing the imaging position of reflected ghost light from a lens surface in the fourth embodiment.

DESCRIPTION OF THE EMBODIMENTS

An exemplary embodiment(s) of the present invention will now be described in detail with reference to the drawings. It should be noted that the relative arrangement of the components, the numerical expressions and numerical values set forth in these embodiments do not limit the scope of the present invention unless it is specifically stated otherwise.

(First Embodiment)

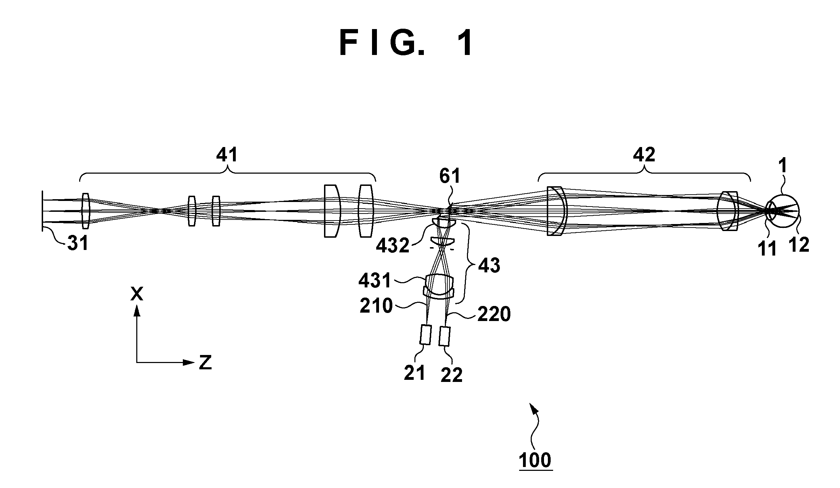

The arrangement of an ophthalmic apparatus (fundus camera 100) according to the first embodiment will be described with reference to Fig. 1. The fundus camera 100 illuminates a retina 12 of an eye 1 to be examined with illumination light from two light sources 21 and 22 through an illumination optical system 43 and an eyepiece optical system 42 (a combination of which will be referred to as an illumination beam optical system (first optical system) hereinafter). This apparatus has an arrangement (image generation processing) configured to acquire a fundus image (two-dimensional image) by forming reflected/backscattered light as return light from the retina 12 into an image on an imaging element 31 through the eyepiece optical system 42 and an imaging optical system 41 (a combination of which will be referred to as an imaging optical system (second optical system) hereinafter). In this case, the illumination beam optical system and the imaging optical system have a common portion (eyepiece optical system 42). Fig. 1 shows an x-z sectional view taken when the optical axis direction is defined as the z-axis, and a direction perpendicular to the z-axis is defined as the x-axis.

The light sources 21 and 22 each are a semiconductor laser which generates light with a wavelength of 780 nm, and are arranged on an x-z section at a predetermined distance from each other. These light sources emit illumination light 210 and illumination light 220. The illumination light 210 and illumination light 220 propagate parallel to the optical axis of the illumination optical system 43 and enter a collimator optical system 431. The collimator optical system 431 collimates the incident light. A lens 432 further converges the light and makes the light propagate almost parallel to the optical axis of the illumination optical system 43 and strike a perforated mirror 61. The illumination light 210 and illumination light 220 are focused once at positions near the perforated mirror 61.

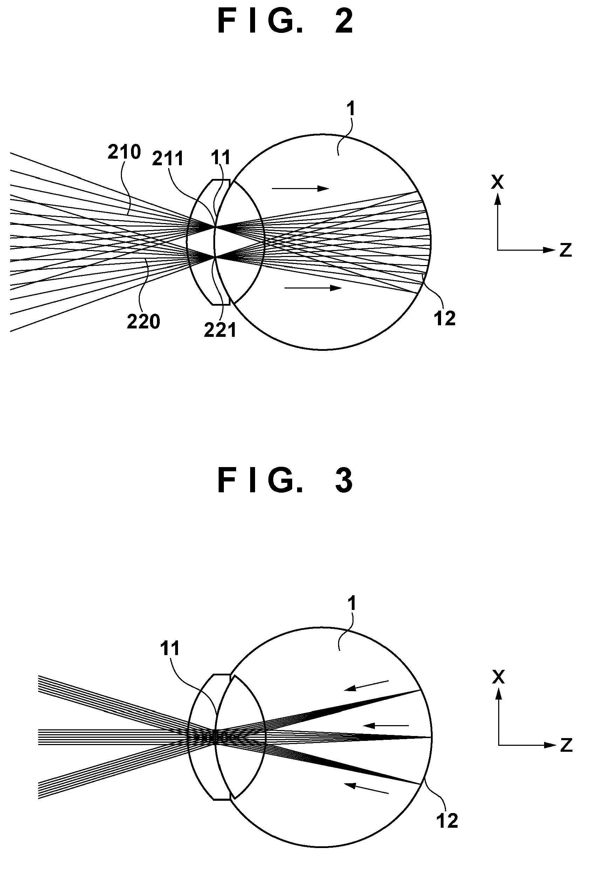

The perforated mirror 61 has an aperture formed near the optical axis of the imaging optical system and a mirror portion formed around the aperture. Each illumination light beam emerging from the illumination optical system 43 is reflected by the mirror portion of the perforated mirror 61 and enters the eyepiece optical system 42. The eyepiece optical system 42 is disposed such that the perforated mirror 61 is optically conjugate to a pupil 11 of the eye 1. The illumination light 210 and illumination light 220 propagating in the eyepiece optical system 42 are focused again at different positions 211 and 221 near the pupil 11, as shown in Fig. 2, which is an enlarged view of a portion of the eye to be examined in Fig. 1. In addition, the illumination light 210 and illumination light 220 enter the eye 1 with their principal rays becoming parallel to the optical axis (visual axis) of the eyepiece optical system 42.

The incident illumination light 210 and illumination light 220 propagate from the pupil 11 and illuminate the retina 12 through the crystalline lens and the corpus vitreum. At this time, the illumination light 210 and illumination light 220 become diverging light after the pupil 11 and illuminate the same region on the retina 12 while almost overlapping each other.

Fig. 3 is an x-z sectional view of the eye 1, which shows return light from the retina 12. Each incident illumination light beam is reflected/backscattered at each position on the retina 12, and emerges as return light from the pupil 11 upon reversely propagating through the corpus vitreum, crystalline lens, and cornea. Each illumination light beam then propagates through the eyepiece optical system 42, the perforated mirror 61, and the imaging optical system 41, and is formed into an image on the imaging element 31. A personal computer (not shown) generates a two-dimensional front image of the retina based on the intensity of the return light received by the imaging element 31. For the sake of viewability, Fig. 3 shows only the light beams from three points on the retina 12. At this time, the aperture of the perforated mirror 61 limits reflected/backscattered light beams from the retina 12 so as to determine the NA (Numerical Aperture) of the overall imaging optical system.

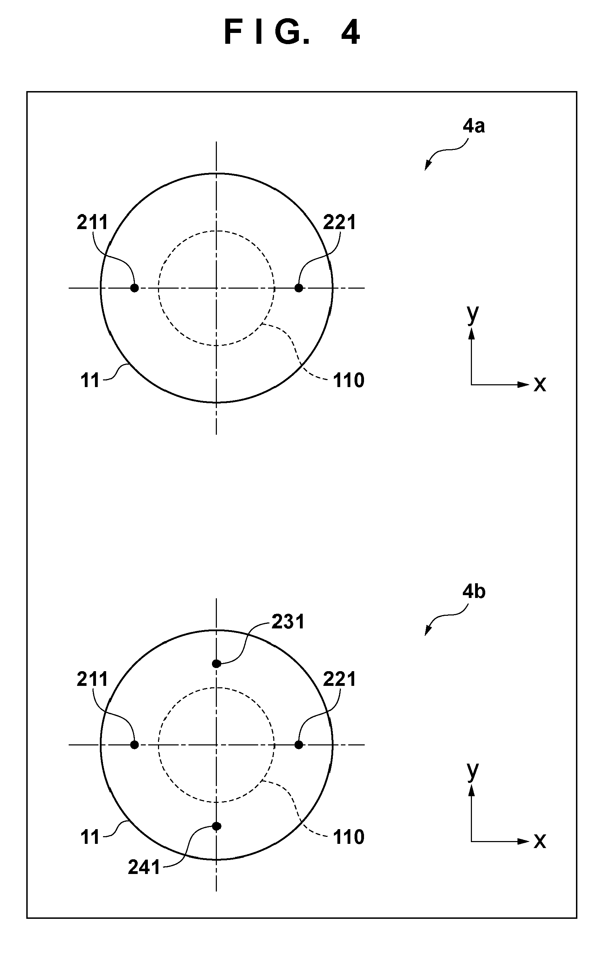

Fig. 4 shows the incident position of each illumination light beam (on an x-y section of the anterior ocular segment) when the anterior ocular segment of the eye 1 in Fig. 1 is viewed from the visual axis (z-axis) direction. Even with individual differences, the pupil 11 is often a circle having a diameter of about 4 mm under normal brightness. The incident positions 211 and 221 are the incident positions of the respective illumination light beams which have entered the pupil. This indicates that measurement light beams are focused near the pupil. The region enclosed by the broke line circle is an effective pupil 110 of the imaging optical system, and is determined by the diameter of the perforated mirror 61. In this case, the diameter is 2 mm on the pupil.

On the other hand, the incident positions 211 and 221 of illumination light beams are set to make the illumination light beams strike outside the effective pupil 110 of the imaging optical system in the pupil 11. In this case, each position is spaced apart from the visual axis by 1.5 mm. Dividing the pupils of the illumination beam optical system and imaging optical system in this manner can remove reflected light from the cornea surface.

In this embodiment, the region on the retina 12 which is illuminated with illumination light has a diameter of about 9 mm. Assume that beam intensities have a Gaussian distribution. In this case, to ensure the evenness of brightness in the irradiation region, it is necessary to focus a beam to several $\mu$m on the pupil. For example, to ensure relative intensity on a peripheral portion around the center of an irradiation region with a diameter of 9 mm up to 60%, the spot diameter on the pupil needs to be about 3.5 $\mu$m.

When ensuring sufficient brightness of a captured image with one illumination light beam, the energy per area on the pupil becomes large. When using light with a near infrared wavelength, this energy generates heat and may strain the tissue such as the cornea or crystalline lens. If two illumination light beams are made to strike at separate positions on the pupil as in this embodiment to avoid such strain, it is possible to ensure double the amount of illumination light on the retina without increasing the strain on the eye 1.

Although this embodiment uses two illumination light beams, it is possible to increase the number of light sources, as shown in Fig. 4. For example, arranging incident positions 231 and 241 as shown in Fig. 4 and further making illumination light beams strike at the positions can increase the brightness of the image four times without increasing the strain on the anterior ocular segment in terms of the amount of each illumination light beam. This makes it possible to expect a further improvement in image quality.

When the anterior ocular segment of the eye 1 has become partially cloudy due to a disease, it is possible to prevent a loss of the amount of illumination light on the retina by providing a mechanism which can individually set the amount of each measurement light beam, and adjusting the amount of each illumination light beam. For example, in normal times, this mechanism sets the amount of each measurement light beam to a relatively small amount. When a cloudy portion exists near the incident position 211 of measurement light to decrease the efficiency of arrival of a corresponding beam to the retina, the mechanism turns off the beam at the incident position 211. Increasing the amounts of beams at the remaining incident positions 221, 231, and 241 can ensure a bright image without decreasing the amount of light illuminating the retina.

Using an illumination light source higher in coherence than spontaneously emitted light, such as a semiconductor laser, will cause speckle noise in a captured image due to the roughness of the surface of the retina. In contrast to this, superimposing illumination light beams from a plurality of light sources as in this embodiment can reduce the such speckle noise. If speckle patterns on a captured image due to four illumination light beams have no correlation with each other, it is possible to reduce the speckle contrast to $1/\sqrt{4}$. Although it is difficult to completely eliminate the correlation, it is possible, according to this embodiment, to reduce the correlation and hence the speckle contrast by making the respective illumination light beams have different polarizations since the incident angles of the illumination light beams on the retina are different.

(Second Embodiment)

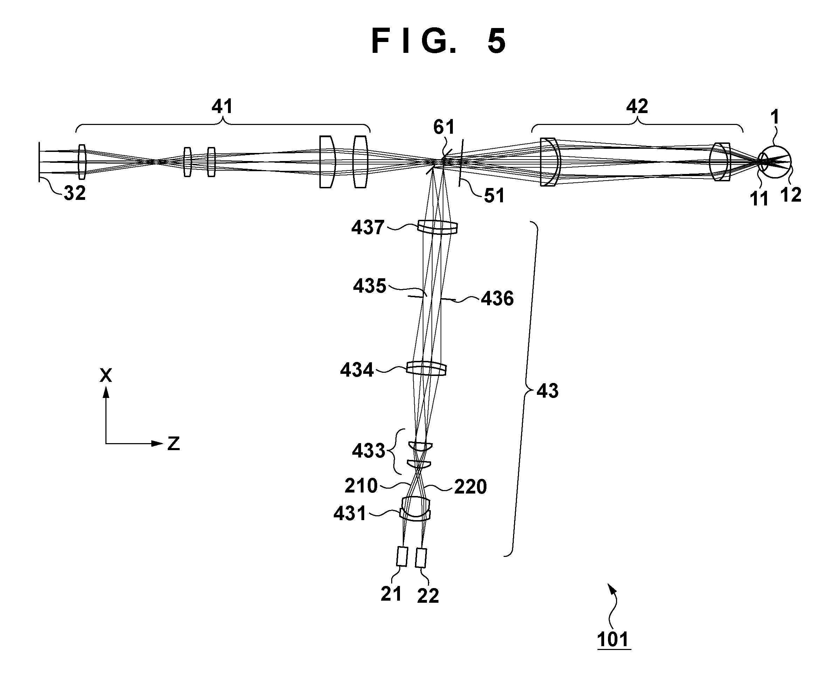

An ophthalmic apparatus (SLO 101) according to the second embodiment will be described next with reference to Fig. 5. The SLO 101 illuminates a retina 12 with linear illumination light, and scans the light in a direction perpendicular to the linear direction. This apparatus is configured to acquire a two-dimensional image by measuring reflected/backscattered light as return light using a light-receiving sensor including a plurality of light-receiving elements arranged in at least one-dimensional direction in the form of a matrix. The coordinates in this case are the same as those in Fig. 1, and linear illumination on the retina 12 coincides with an x-z section.

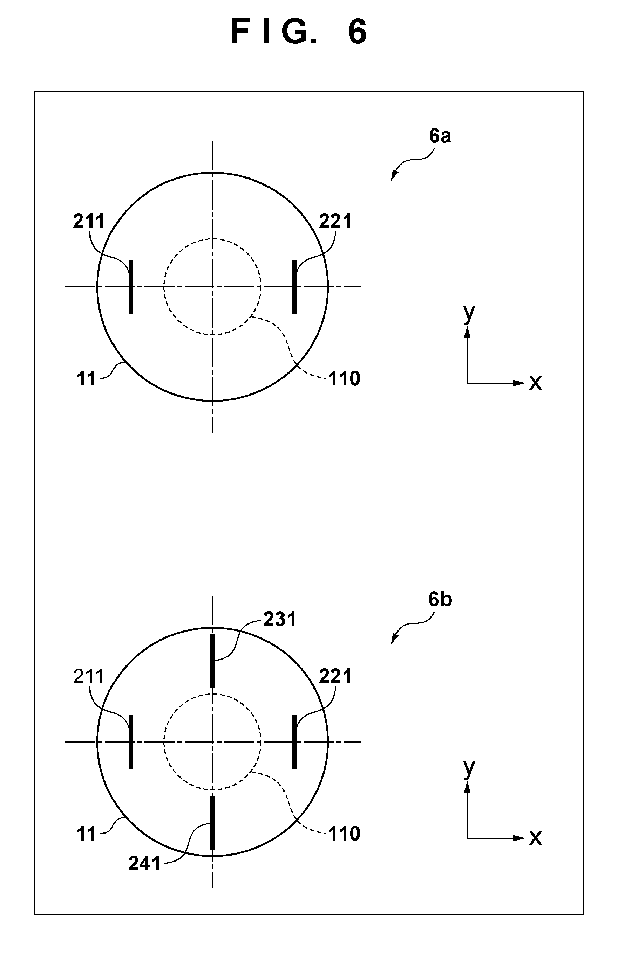

In order to form such illumination light, illumination light propagating to the retina 12 needs to have the property of diverging in the linear direction (x direction) of the linear illumination on the retina 12 and focusing in a direction perpendicular to the linear direction (a direction in which illumination light is scanned: y direction). Therefore, the illumination light must be a beam which is focused in the x direction and is almost parallel to the y direction if the diopter of an eye 1 to be examined is 0 D. In this embodiment, an illumination region (image acquisition region) on the retina 12 was set to 9 mm (x direction) x 6 mm (y direction). In this case, as in the first embodiment, since the beam diameter in the x direction near the pupil is set to about 3.5 μm, two illumination light beams enter from different positions on a pupil 11 of the eye 1 to reduce the strain on the anterior ocular segment to half and reduce speckle noise. Fig. 6A shows the arrangement of incident positions of illumination light beams on the pupil 11 within an x-y plane.

Each reference numeral is the same as that in Fig. 4. Incident positions 211 and 221 of two illumination light beams each are spaced apart from the pupil center by 1.5 mm so as to be parallel to each other. Each illumination light beam is focused to a diameter of 3.5 μm only in the x direction and becomes a linear beam having a length of 9 mm on the retina 12. Each illumination light beam is a parallel beam having a size of about 1 mm in the y direction and is focused to a width of about 20 μm on the retina 12. The illumination regions of the two formed linear illumination light beams almost coincide with each other on the retina 12. A single scanner mirror 51 simultaneously scans these illumination light beams in the y direction (perpendicular to the drawing surface in Fig. 5) to illuminate a predetermined region.

As shown in Fig. 5, the basic arrangement of an optical system includes an illumination optical system 43, an eyepiece optical system 42, and an imaging optical system 41, as in the first embodiment. In the illumination optical system 43, first of all, a collimator optical system 431 collimates illumination light emitted as diverging light from light sources 21 and 22. At this time, the principal ray of each illumination light beam entering the collimator optical system 431 is parallel to the optical axis of the illumination optical system 43.

A cylindrical lens unit 433 including optical elements having different optical powers (optical characteristics) on a sagittal section and a meridional section focuses each of the collimated illumination light beams in only the x direction. At this time, the principal ray of light emerging from the cylindrical lens unit 433 is almost parallel to the optical axis of the illumination optical system 43. A lens 434 focuses each illumination light beam in the y direction. This illumination light beam becomes a parallel beam in the x direction to form an intermediate image 435. This position is optically conjugate to the retina 12, at which the respective illumination light beams almost coincide with each other. In this embodiment, an aperture 436 is set at this position to define the length of linear illumination light on the retina 12, and shields an unnecessary portion of each illumination light beam.

Illumination light passing through the aperture 436 is collimated by a lens 437 in the y direction, and is focused near a perforated mirror 61 in the x direction. The perforated mirror 61 is disposed at a position optically conjugate to the pupil 11 of the eye 1. A scanner mirror 51 disposed near the perforated mirror 61 reflects and scans each illumination light beam reflected by the perforated mirror 61 in only the y direction. This light beam then becomes a parallel beam in the y direction at a position near the pupil through the eyepiece optical system 42, and is focused in the x direction, thus becoming linear illumination light.

Subsequently, the return light reflected/backscattered by the retina 12 passes through the aperture of the perforated mirror 61 through the eyepiece optical system 42 and the scanner mirror 51, and is formed into a linear image on a line camera 32 through the imaging optical system 41. Note that although the eyepiece optical system 42 and the eye 1 are actually arranged in a direction (y direction) perpendicular to the drawing surface, and the scanner mirror 51 reflects light almost vertically, they are drawn in the same plane for the sake of viewability in Fig. 5. The scanner mirror 51 rotates through a small angle about the line in Fig. 5 as a rotation axis to scan linear illumination light in the y direction on the retina. Note that a personal computer (not shown) controls the scanner mirror 51 and generates a two-dimensional planar image on the retina 12 from the intensity of return light obtained by the line camera 32 in synchronism with the operation of the scanner mirror 51.

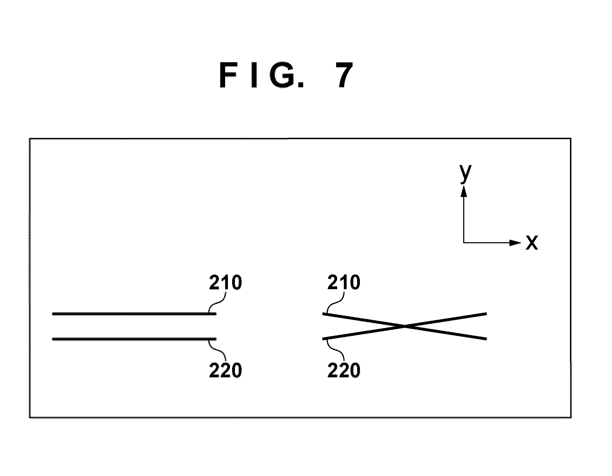

Assume that a straight line connecting the two light sources 21 and 22 is not parallel to the generator of the cylindrical surface of the cylindrical lens unit 433 but has some angle due to a manufacturing error or the like, and the aberration of the eye 1 is large. In this case, as shown in Fig. 7, two linear illumination light beams 210 and 220 are spaced apart from each other on the retina 12 or have an angle and do not overlap. In such a case, since the reflected/backscattered light from the retina 12 is not efficiently formed into an image on the light-receiving unit of the line camera 32, the acquired image becomes dark. In some case, the resolution of the image decreases in the scanning direction.

To prevent this phenomenon, this apparatus may have a fine adjusting mechanism which allows the light source unit including the two light sources 21 and 22 or the cylindrical lens unit 433 to rotate about the optical axis of the illumination optical system 43. For example, the user may visually adjust two linear illumination light beams so as to superimpose them on the retina 12, or the apparatus may be configured to automatically adjust the two linear illumination light beams upon determining, by detecting the light amount in each region, whether they are properly superimposed. This makes it possible to always properly superimpose two linear illumination light beams on the retina 12 and stably obtain a bright image.

Note that in order to form linear illumination light, a toric lens, a diffraction optical element, or the like may be used instead of the cylindrical lens.

As described above, even in a line SLO designed to acquire an image by scanning linear illumination light, using the arrangement of this embodiment can ensure a bright image and reduce the speckle contrast while suppressing strain on the anterior ocular segment.

As shown in Fig. 6B, in addition, in this embodiment as well, making the two illumination light beams 231 and 241 further enter the anterior ocular segment can further improve the above effects.

(Third Embodiment)

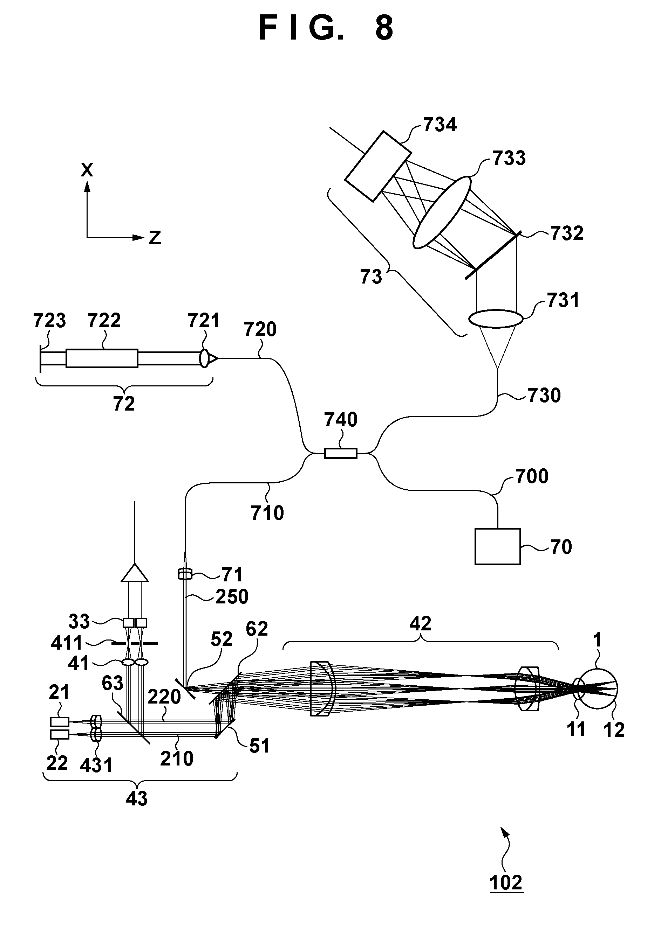

The arrangement of an ophthalmic apparatus according to the third embodiment will be described with reference to Fig. 8. This embodiment exemplifies an ophthalmic imaging apparatus 102 including both an OCT and an SLO. As in the first and second embodiments, the SLO includes an illumination optical system 43, an eyepiece optical system 42, and an imaging optical system 41. In addition, the OCT is based on a spectral domain (SD) scheme in which an interferometer is constituted by a light source 70, a sample optical system sharing the eyepiece optical system 42 with the SLO, a reference optical system 72, and a spectroscope 73.

Both the SLO and the OCT in this embodiment use a scheme of two-dimensionally scanning a small spot on the retina in the vertical and horizontal directions. The SLO allows the operator to simultaneously observe an OCT tomogram (B-scan image) and a front image in association with each other while monitoring the front image of a wide region of the retina. Obtaining a front image of the retina at a high frame rate can perform tracking operation reflected in an acquisition region of the OCT by using a feature point on the image and calculating the movement of the eye.

The OCT unit of this embodiment will be described first. Light emitted from the light source 70, which has a central wavelength of 850 nm, a spectral width of 50 nm, and low coherence, propagates in a single mode fiber 700, and is split at a proper ratio by a coupler 740. The split beams then propagate to fibers 710 and 720.

The light which has propagated in the fiber 710 emerges as diverging light from the fiber end. A collimator lens 71 then collimates this light into measurement light 250, which strikes a scanner mirror 52. The measurement light 250 is reflected/deflected by the scanner mirror 52 and is transmitted through a dichroic mirror 62. The light then enters an eye 1 to be examined through the eyepiece optical system 42 and is scanned on a retina 12.

The scanned measurement light 250 is reflected/backscattered by the retina 12, reversely propagates through the anterior ocular segment of eye 1, the eyepiece optical system 42, the scanner mirror 52, and the collimator lens 71, and enters the fiber 710, thus propagating to the coupler 740. Note that a personal computer (not show) controls the scanner mirror 52.

The light which has propagated in the fiber 720 emerges as diverging light from the fiber end. This light propagates through a collimator lens 721 and a dispersion-compensating glass 722 and is reflected by a folding mirror 723. The reflected light reversely propagates through the dispersion-compensating glass 722 and the collimator lens 721, enters the fiber 720, and propagates to the coupler 740.

The light beams which have reversely propagated in the fibers 710 and 720, respectively, propagate through a fiber 730 from the coupler 740. The light then emerges as diverging light from the fiber end. A collimator lens 731 collimates the light. A grating 732 diffracts the collimated light at different angles for the respective wavelengths. An imaging lens 733 forms the light into an image on a line camera 734.

The fringe pattern acquired by the line camera 734 is output to a personal computer (not shown). The personal computer converts the wavelength into a wave number and then performs Fourier transform, thereby calculating information (A-scan image) in the depth direction of the retina. Imaging this information in correspondence with beam scanning will obtain a tomogram (B-scan image).

The arrangement of the SLO provided together with the OCT will be described next. As in the first and second embodiments, a light source is constituted by two light sources 21 and 22 each having a central wavelength of 780 nm. Both the light sources emit diverging light parallel to the optical axis of the illumination optical system 43. These light beams enter the collimator optical system 431 constituted by two collimator lenses made to correspond to the respective illumination light beams and are collimated.

Collimated illumination light beams 210 and 220 are transmitted through a half mirror 63 and then are deflected in a two-dimensional direction by a scanner mirror 51. These light beams are reflected by a dichroic mirror 62 and enter the eye 1 through the eyepiece optical system 42 shared with the OCT. The two illumination light beams which have entered the eye 1 pass through a pupil 11 and are two-dimensionally scanned on the retina 12. The two reflected/ backscattered light beams from the retina 12 are reversely transmitted through the anterior ocular segment, the pupil 11, and the eyepiece optical system 42, reflected by the dichroic mirror 62, and strike the scanner mirror 51. Note that the personal computer controls the scanner mirror 51, like the scanner mirror 52 of the OCT.

The return light beams scanned by the scanner mirror 51 are reflected by the half mirror 63 and are focused to two pinholes 411 through the imaging optical system 41. The light beams pass through the hole portions and enter two photodetectors 33. At this time, the pinholes 411 are disposed in a positional relationship optically conjugate to the retina. The detected two light beams are converted into electrical signals and added. The resultant data is sent to the personal computer. The personal computer acquires the electrical signal obtained here in synchronism with beam scanning, thereby obtaining a two-dimensional front retinal image.

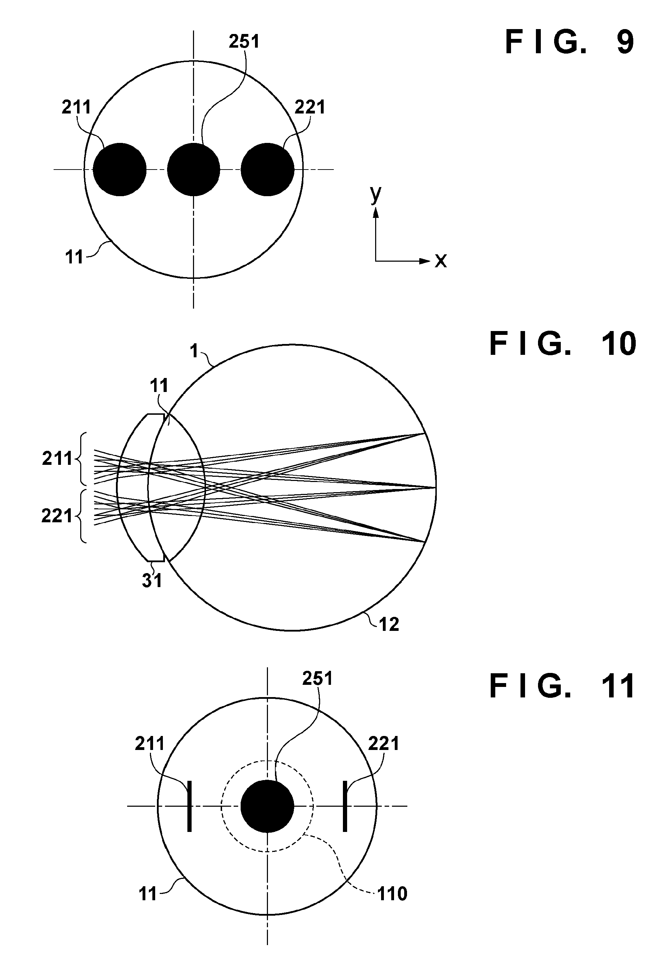

Fig. 9 shows the arrangement of the respective incident beams on an x-y plane at this time. OCT illumination light 251 is guided to almost the center of the pupil 11 with a diameter of 4 mm. Two SLO illumination light 211 and illumination light 221 are guided to separate positions located on the two sides of the OCT illumination light 251 so as not to overlap each other. The three illumination light beams each have a diameter of 1 mm, and the intervals between the principal rays of the respective illumination light beams are 1.25 mm.

Fig. 10 is an x-z sectional view of the eye 1 in Fig. 8, and shows only the two SLO illumination light 211 and illumination light 221 for the sake of viewability. The interval between the principal rays of the two illumination light beams on the pupil is 2.5 mm. On the retina, the two beam spots are scanned while being superimposed. In general, the imaging speed of the SLO is often higher than that of the OCT, and scans are not synchronized. For this reason, SLO and OCT beam spots are not frequently superimposed on the retina.

The above arrangement allows to obtain the effects of reducing strain on the anterior ocular segment, reducing speckle noise, and performing imaging outside a morbid portion of the anterior ocular segment even in an ophthalmic apparatus having a plurality of functions. Note that it is possible to obtain the same effects as those described above even by using the line SLO in the second embodiment. Fig. 11 shows the arrangement of the respective incident beams on the pupil in this case. The three illumination light beams, namely the OCT illumination light 251, the SLO illumination light 211, and the SLO illumination light 221, strike the pupil 11. Note that a pupil 110 is the pupil of the line SLO which is determined by the diameter of the perforated mirror.

(Fourth Embodiment)

When using the arrangement of the optical system like that disclosed in the first to third embodiments, actual return light from the fundus is often weak. For this reason, the fourth embodiment attempts to acquire a better fundus image by properly removing reflected light from each lens surface.

The reflectance of the fundus is on the order of $10^{-5}$ to $10^{-4}$, whereas the reflectance of a lens surface is often on the order of about $10^{-4}$ to $10^{-3}$ even when an antireflection film is formed on the surface. Therefore, when reflected light from the lens surface reaches an imaging area of the light-receiving surface of the light-receiving sensor, the light becomes a signal having an intensity equal to or several times higher than that of return light from the fundus. This signal is detected as a reflected ghost image, which hinders from acquiring a good image.

As a method of removing reflected ghost light in a fundus camera or the like, there is known a method of shielding against reflected ghost light by placing a minute black point midway along the optical path of an illumination optical system. There is also conceivable a method of setting an optical system to make reflected ghost light strike a light-receiving sensor, upon becoming a diverging beam which widely diverges, and reducing the amount of light per area when it is received.

However, using a black point will also shield against part of return light from the retina as well as reflected ghost light. As a consequence, an acquired image becomes partially dark. As the number of lens surfaces increases, it is necessary to increase the number of black points. This leads to a dark image and the loss of brightness evenness. In addition, unlike the case of lamp illumination, the influence of this phenomenon further increases on a scheme of scanning a thin beam.

The method of making reflected ghost light strike the light-receiving sensor upon diverging is difficult to apply to reflected ghost light from all the surfaces while satisfying the requirement for the imaging performance of return light from the retina.

In the first to third embodiments, an illumination beam is set to enter the eyeball while shifting relative to the visual axis. If, therefore, an eyepiece optical system is set to be coaxial with the visual axis, the illumination light also shifts from the optical axis of the lens. Therefore, properly setting the optical system can focus reflected ghost light to the outside of the operation area of the light-receiving sensor.

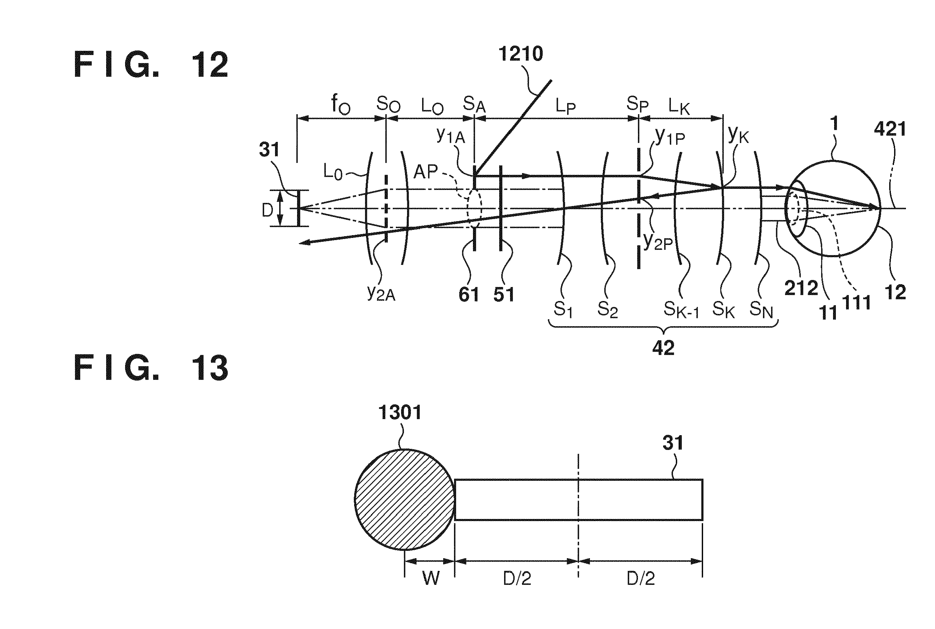

Assume that the common portion between the first and second optical systems has an arrangement like that shown in Fig. 12. One light beam (its principal ray) 1210 of beams from illumination light sources strikes and is reflected by the mirror portion of a perforated mirror 61 having a function of limiting return light from the retina. In this case, an aperture AP of the perforated mirror is disposed at some angle so as to match the optical axis of the common portion between the first and second optical systems. For the sake of viewability, however, Fig. 12 shows the aperture AP without any tilt.

The reflected beam 1210 is reflected by a one-dimensional scanner 51 adjacent to the perforated mirror, strikes a pupil 11 of an eyeball 1 through an eyepiece optical system 42 constituted by lens surfaces $S_1$ to $S_N$, and linearly illuminates a retina 12. Reflected/backscattered light 1212 from the retina 12 emerges from the pupil 11 and reversely propagates through the eyepiece optical system 42 and the aperture AP of the perforated mirror 61. An imaging optical system 41 then forms this light into an image on a linear light-receiving sensor 31. Consider reflected ghost light generated by the lens surface $S_k$ ($k \leq N$) where N is an integer equal to or more than 2.

Light transmitted through the lens surfaces $S_1$ to $S_{k-1}$ of the eyepiece optical system 42 is partially reflected by the lens surface $S_k$, and is reversely transmitted through the lens surfaces $S_{k-1}$ to $S_1$ to reach the perforated mirror 61. In this case, if the reflected light is entirely shielded by the mirror portion, the light-receiving sensor 31 detects no reflected ghost light. If, however, the reflected light entirely or partially passes through the aperture AP depending on conditions, the imaging optical system 41 forms the light into an image on the light-receiving sensor 31. As a result, a strong ghost image appears on the image.

Even in this case, no ghost image appears on the image if the imaging position of the reflected ghost light on the surface of the light-receiving sensor 31 is made to reach outside an area used as a range used for the reception of light by adjusting conditions such as the curvature of each surface of the eyepiece optical system 42 and inter-surface distances.

Specific conditions for this purpose will be described below. Let $y_{1A}$ be the ray height of the principal ray of the illumination beam 1210 at a reflection point on the perforated mirror 61 from an optical axis 421 of the eyepiece optical system 42, and $u_{1A}$ be the ray angle of the reflected beam (assuming that the counterclockwise direction is positive). In addition, letting $f_{P(k-1)}$ be the composite focal length of the lens surfaces $S_1$ to $S_{k-1}$, Sp be a principal surface position, Lp be the distance from a light beam limiting unit $S_A$ which limits a return light beam from the eye to be examined to the principal surface position Sp, a ray height $y_{1P}$ on the principal surface position Sp and an exit ray angle $u_{1k}$ relative to the optical axis 421 from the lens surface $S_{k-1}$ are expressed as follows according to paraxial calculation:

$$y_{1P} = y_{1A} - L_P \cdot u_{1P} \qquad \ldots (1-1)$$

$$u_{1k} = y_{1P}/f_{P(k-1)} + u_{1P}$$
$$= (y_{1A} - L_P \cdot u_{1P})/f_{P(k-1)} + u_{1P}$$
$$= y_{1A}/f_{P(k-1)} + (1 - L_P/f_{P(k-1)}) \cdot u_{1P} \qquad \ldots (1-2)$$

Likewise, according to equations (1-1) and (1-2), letting $L_k$ be the distance from the principal surface position Sp to the lens surface $S_k$ and $R_k$ be the curvature radius of the lens surface $S_k$, a ray height $y_k$ on the lens surface $S_k$ and a reflected light angle $U_{2k}$ from the lens surface $S_k$ are expressed as follows:

$$y_k = y_{1P} - L_k \cdot u_{1k}$$
$$= y_{1P} - L_k \cdot (y_{1P}/f_{P(k-1)} + u_{1P})$$
$$= (1 - L_k/f_{P(k-1)}) \cdot y_{1P} - L_k \cdot u_{1P}$$
$$= (1 - L_k/f_{P(k-1)}) \cdot (y_{1A} - L_P \cdot u_{1P}) - L_k \cdot u_{1P}$$
$$= (1 - L_k/f_{P(k-1)}) \cdot y_{1A} - (1 - L_k/f_{P(k-1)} + L_k) \cdot L_P \cdot u_{1P}$$

$$\ldots (2-1)$$

$$u_{2k} = 2 \cdot y_k/R_k + u_{1k}$$

$$= 2 \cdot \{(1-L_k/f_{P(k-1)}) \cdot y_{1A} - (1-L_k/f_{P(k-1)} + L_k) \cdot L_P \cdot u_{1P}\}/R_k + y_{1A}/f_{P(k-1)} + (1-L_P/f_{P(k-1)}) \cdot u_{1P}$$

$$= \{2 \cdot (1-L_k/f_{P(k-1)})/R_k + 1/{}_{fP(k-1)}\} \cdot y_{1A} + \{-2 \cdot (1-L_k/f_{P(k-1)} + L_k) \cdot L_P/R_k + (1-L_P/f_{P(k-1)})\} \cdot u_{1P}$$

$$\ldots (2-2)$$

Likewise, according to equations (2-1) and (2-2), a ray height $Y_{2P}$ on the principal surface position Sp and an exit light angle $U_{2P}$ from the lens surface $S_1$ are from the lens surface $S_k$ are expressed as follows:

$$y_{2P} = y_k - L_k \cdot u_{2k}$$

$$= y_k - L_k \cdot (2 \cdot y_k/R_k + u_{1k})$$

$$= y_k - 2 \cdot L_k/R_k \cdot y_k - L_k \cdot u_{1k}$$

$$= (1 - 2 \cdot L_k/R_k) \cdot y_k - L_k \cdot u_{1k}$$

$$= (1 - 2 \cdot L_k/R_k) \cdot \{(1-L_k/f_{P(k-1)}) \cdot y_{1A} - (1-L_k/f_{P(k-1)} + L_k) \cdot L_P \cdot u_{1P}\} - L_k \cdot \{y_{1A}/f_{P(k-1)} + (1-L_P/f_{P(k-1)}) \cdot u_{1P}\}$$

$$= (1 - 2 \cdot L_k/R_k) \cdot (1-L_k/f_{P(k-1)}) \cdot y_{1A} - (1 - 2 \cdot L_k/R_k) \cdot (1-L_k/f_{P(k-1)} + L_k) \cdot L_P \cdot u_{1P} - L_k/f_{P(k-1)} \cdot y_{1A} - L_k \cdot (1-L_P/f_{P(k-1)}) \cdot u_{1P}$$

$$= \{(1 - 2 \cdot L_k/R_k) \cdot (1-L_k/f_{P(k-1)}) - L_k/f_{P(k-1)}\} \cdot y_{1A} + \{-(1 - 2 \cdot L_k/R_k) \cdot (1-L_k/f_{P(k-1)} + L_k) \cdot L_P - L_k/f_{P(k-1)}\} \cdot u_{1P}$$

$$\ldots (3-1)$$

$$u_{2P} = y_{2P}/f_{P(k-1)} + u_{2k}$$

$$= [\{(1 - 2 \cdot L_k/R_k) \cdot (1-L_k/f_{P(k-1)}) - L_k/f_{P(k-1)}\}$$

$$\cdot y_{1A} + \{-(1 - 2 \cdot L_k/R_k) \cdot (1-L_k/f_{P(k-1)} + L_k) \cdot L_P - L_k$$

$$/f_{P(k-1)}\} \cdot u_{1P}]/f_{P(k-1)} + \{2 \cdot (1-L_k/f_{P(k-1)})/R_k$$

$$+ 1/f_{P(k-1)}\} \cdot y_{1A} + \{-2 \cdot (1-L_k/f_{P(k-1)} + L_k) \cdot L_P/R_k$$

$$+ (1-L_P/f_{P(k-1)})\} \cdot u_{1P}$$

$$= [\{(1 - 2 \cdot L_k/R_k) \cdot (1-L_k/f_{P(k-1)}) - L_k/f_{P(k-1)}\}$$

$$/f_{P(k-1)} + \{2 \cdot (1-L_k/f_{P(k-1)})/R_k + 1/f_{P(k-1)}\}]$$

$$\cdot y_{1A} + [\{-(1 - 2 \cdot L_k/R_k) \cdot (1-L_k/f_{P(k-1)} + L_k)$$

$$\cdot L_P - L_k/f_{P(k-1)}\}/f_{P(k-1)} + \{-2 \cdot (1-L_k/f_{P(k-1)} + L_k)$$

$$\cdot L_P/R_k + (1-L_P/f_{P(k-1)})\}] \cdot u_{1P}$$

$$\ldots (3-2)$$

Subsequently, according to equations (3-1) and (3-2), letting $L_0$ be the distance from the perforated mirror 61 to a principal surface $S_0$ of the imaging optical system 41 and $f_0$ be the focal length of the imaging optical system 41, a ray height $y_0$ on $S_0$ and an exit light angle $u_0$ from $S_0$ are expressed as follows:

$$y_0 = y_{2P} - (L_P+L_0) \cdot u_{2P}$$

$$= y_{2A} - (L_P+L_0) \cdot (y_{2P}/f_{P(k-1)} + u_{2k})$$

$$= y_{2P} - (L_P+L_0)/f_{P(k-1)} \cdot y_{2P} - (L_P+L_0) \cdot u_{2k}$$

$$= \{1 - (L_P+L_0)/f_{P(k-1)}\} \cdot y_{2P} - (L_P+L_0) \cdot u_{2k}$$

$$= \{1 - (L_P+L_0)/f_{P(k-1)}\} \cdot [\{(1 - 2 \cdot L_k/R_k)$$

$$\cdot (1-L_k/f_{P(k-1)}) - L_k/f_{P(k-1)}\} \cdot y_{1A} - (1 - 2 \cdot L_k/R_k)$$

$$\cdot (1-L_k/f_{P(k-1)} + L_k) \cdot L_P - L_k/f_{P(k-1)}\} \cdot u_{1P}]$$

$$- (L_P+L_0) \cdot [\{2 \cdot (1-L_k/f_{P(k-1)})/R_k + 1/f_{P(k-1)}\}$$

$$\cdot y_{1A} + \{-2 \cdot (1-L_k/f_{P(k-1)} + L_k) \cdot L_P/R_k$$

$$+ (1-L_P/f_{P(k-1)})\} \cdot u_{1P}]$$

$$= [\{1 - (L_P+L_0)/f_{P(k-1)}\} \cdot \{(1 - 2 \cdot L_k/R_k)$$

$$\cdot (1-L_k/f_{P(k-1)}) - L_k/f_{P(k-1)}\} - (L_P+L_0)$$

$$\cdot \{2 \cdot (1-L_k/f_{P(k-1)})/R_k + 1/f_{P(k-1)}\}]$$

$$\cdot y_{1A} + [\{1 - (L_P+L_0)/f_{P(k-1)}\} \cdot \{-(1 - 2 \cdot L_k/R_k)$$

$$\cdot (1-L_k/f_{P(k-1)} + L_k) \cdot L_P - L_k/f_{P(k-1)}\} - (L_P+L_0)$$

$$\cdot \{-2 \cdot (1-L_k/f_{P(k-1)} + L_k) \cdot L_P/R_k$$

$$+ (1-L_P/f_{P(k-1)})\}] \cdot u_{1P}$$

$$\cdots(4-1)$$

$$u_0 = y_0/f_0 + u_{2P}$$

$$= [[\{1 - (L_P+L_0)/f_{P(k-1)}\} \cdot \{(1 - 2 \cdot L_k/R_k)$$

$$\cdot (1-L_k/f_{P(k-1)}) - L_k/f_{P(k-1)}\} - (L_P+L_0) \cdot \{2 \cdot (1$$

$$- L_k/f_{P(k-1)})/R_k + 1/f_{P(k-1)}\}]\cdot y_{1A} + [\{1 -$$

$$(L_P+L_0)/f_{P(k-1)}\}\cdot\{-(1 - 2\cdot L_k/R_k)\cdot(1-L_k$$

$$/f_{P(k-1)} + L_k)\cdot L_P - L_k /f_{P(k-1)}\} - (L_P+L_0)\cdot\{-2$$

$$\cdot(1-L_k/f_{P(k-1)}+L_k)\cdot L_P/R_k+(1-L_P/f_{P(k-1)})\}]$$

$$\cdot u_{1P}]/f_0 + [\{(1 - 2\cdot L_k/R_k)\cdot(1-L_k/f_{P(k-1)})$$

$$- L_k/f_{P(k-1)}\}/f_{P(k-1)} + \{2\cdot(1-L_k/f_{P(k-1)})/R_k$$

$$+ 1/f_{P(k-1)}\}]\cdot y_{1A} + [\{-(1 - 2\cdot L_k/R_k)\cdot(1-L_k$$

$$/f_{P(k-1)} + L_k)\cdot L_P - L_k/f_{P(k-1)}\}/f_{P(k-1)} + \{-2\cdot(1-L_k$$

$$/f_{P(k-1)} + L_k)\cdot L_P/R_k + (1-L_P/f_{P(k-1)})\}]\cdot u_{1P}$$

$$...(4-2)$$

Therefore, an imaging position $y_{im}$ of reflected ghost light on the light-receiving sensor 31 is given bv

$$y_{im} = y_0 - f_0\cdot u_0$$

$$= y_0 - f_0\cdot(y_0/f_0 + u_{2P})$$

$$= y_0 - y_0 - f_0\cdot u_{2P}$$

$$= -f_0\cdot u_{2P}$$

$$= -f_0\cdot[\{(1- 2\cdot L_k/R_k)\cdot(1-L_k/f_{P(k-1)}) - L_k$$

$$/f_{P(k-1)}\}/f_{P(k-1)} + \{2\cdot(1-L_k/f_{P(k-1)})/R_k + 1$$

$$/f_{P(k-1)}\}]\cdot y_{1A} + [\{-(1 - 2\cdot L_k/R_k)\cdot(1-L_k$$

$$/f_{P(k-1)} + L_k)\cdot L_P - L_k/f_{P(k-1)}\}/f_{P(k-1)} + \{-2\cdot(1-L_k$$

$$/f_{P(k-1)} + L_k)\cdot L_P/R_k + (1-L_P/f_{P(k-1)})\}]\cdot u_{1P}$$

$$= [-f_0\cdot[\{(1 - 2\cdot L_k/R_k)\cdot(1-L_k/f_{P(k-1)}) - L_k$$

$$/f_{P(k-1)}\}/f_{P(k-1)} + \{2\cdot(1-L_k/f_{P(k-1)})/R_k + 1$$

$$/f_{P(k-1)}\}]]\cdot y_{1A} + [-f_0\cdot[\{-(1 - 2\cdot L_k/R_k)$$

$$\cdot(1-L_k/f_{P(k-1)} + L_k)\cdot L_P - L_k/f_{P(k-1)}\}/f_{P(k-1)} +$$

$$\{-2\cdot(1-L_k/f_{P(k-1)} + L_k)\cdot L_P/R_k + (1-L_P/f_{P(k-1)})\}]]$$

$$\bullet u_{1P} \qquad\qquad \ldots(5\text{-}1)$$

Letting W be the radius (the radius with a relative intensity of $1/e^2$ or less being a threshold) of a reflected ghost light beam on the light-receiving sensor 31 (on the detection surface) and D be the area used for detection by the light-receiving sensor, if

$$y_{im} > D/2 + W \qquad\qquad \ldots(6\text{-}1)$$

then, as shown in Fig. 13 reflected ghost light 1301 reaches outside the image acquisition area of the light-receiving sensor 31 even in consideration of the thickness of the reflected ghost light beam, and hence no reflected ghost image from the lens appears on the image.

Therefore, setting all the lens surfaces $S_1$, $S_2$,..., $S_N$ constituting the eyepiece optical system 42 so as to satisfy inequality (6-1) can obtain a good retinal image without any reflected ghost light appearing on the image.

If it is difficult to satisfy inequality (6-1) with respect to all the surfaces, it is possible to set a black point or divergence conditions for reflected ghost light on a given surface or make settings to satisfy inequality (6-1) on another given surface.

As has been described above, the curvatures, positions, and refractive indices of lens surfaces are set so as to cause at least the principal ray of a reflected illumination light beam reflected by at least one of the surfaces of the lenses constituting the common portion between the first and second optical systems to reach outside the imaging area of the light-receiving element of the light-receiving sensor.

According to the present invention, it is possible to increase the amount of light applied on the retina while alleviating strain on the cornea or crystalline lens by making a plurality of illumination light beams strike the anterior ocular segment upon separating them, and superimposing them on the retina.

(Other Embodiments)

Aspects of the present invention can also be realized by a computer of a system or apparatus (or devices such as a CPU or MPU) that reads out and executes a program recorded on a memory device to perform the functions of the above-described embodiment(s), and by a method, the steps of which are performed by a computer of a system or apparatus by, for example, reading out and executing a program recorded on a memory device to perform the functions of the above-described embodiment(s). For this purpose, the program is provided to the computer for example via a network or from a recording medium of various types serving as the memory device (for example, computer-readable storage medium).

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1. An ophthalmic apparatus comprising:

   a first optical system (42, 43) which irradiates the same region on a retina with a plurality of illumination light beams through separate positions on a pupil of an eye to be examined; and
   image generation means (31) for generating a two-dimensional image of the retina based on return light from the eye.

2. The apparatus according to claim 1, further comprising light-receiving means (31) for receiving the return light through a second optical system (41, 42) sharing a portion with said first optical system (42, 43),
   wherein said image generation means (31) generates a two-dimensional image of the retina based on the return light received by said light-receiving means.

3. The apparatus according to claim 1, wherein said first optical system (42, 43) always illuminates the same region on the retina with the illumination light while coinciding the illumination light with the region at the time of image formation, and

said image generation means (31) generates a two-dimensional image of the retina based on a signal obtained by adding return light beams generated when the respective illumination light beams illuminate the retina.

4. The apparatus according to claim 2, further comprising generation means (21, 22) for generating the plurality of illumination light beams by using a plurality of different light sources.

5. The apparatus according to claim 4, wherein said generation means (21, 22) is configured to individually set light amounts for a plurality of illumination light beams to be made to strike the pupil.

6. The apparatus according to claim 2, wherein said first optical system (42, 43) receives the plurality of illumination light beams from an outside of an effective pupil of a second optical system (41, 42) on the pupil.

7. The apparatus according to claim 2, wherein said first optical system (42, 43) linearly applies the plurality of illumination light beams on the retina.

8. The apparatus according to claim 7, further comprising scanning means (101) for scanning the linear illumination light in a direction perpendicular to a longitudinal direction.

9. The apparatus according to claim 8, wherein said scanning means (101) is disposed at a position optically conjugate to the pupil.

10. The apparatus according to claim 9, wherein said light-receiving means receives return light of the linear illumination light scanned by said scanning means (101) from the retina by causing light-receiving elements arranged in a matrix to form the return light into an image.

11. The apparatus according to claim 1, further comprising limiting means (61, $S_A$) for limiting the illumination light beam to a position optically conjugate to the retina in said first optical system (42, 43).

12. The apparatus according to claim 1, further comprising an adjusting mechanism which is configured to rotate, about an optical axis of said first optical system (42, 43), at least one of a light source unit including a plurality of light sources which generate the plurality of illumination light beams and a unit including an optical elements which are s included in said first optical system (42, 43) and exhibit different optical characteristics on a sagittal section and a meridional section.

13. The apparatus according to claim 2, further comprising an interferometer including a sample optical system including a portion of a common portion between said first optical system (42, 43) and said second optical (41, 42) system, a reference optical system including a folding mirror, a light source which has low coherence and a wavelength different from a light source for the illumination light, and a detector.
a interferometer further comprises light guide means for causing illumination light formed by said light source which has low coherence and a wavelength different from said light source for the illumination light and said sample optical system to enter from a region where the illumination light is separated from the plurality of illumination light beams on the pupil.

14. The apparatus according to claim 13, wherein said interferometer further comprises light guide means for entering illumination light, which is formed by the sample optical system and the light source which has the low coherence and the wavelength different from the light source for the illumination light, from separate positions from the plurality of illumination light beams on the pupil.

15. The apparatus according to claim 14, wherein said interferometer acquires a tomogram by causing said detector to detect return light of the illumination light guided by said light guide means.

16. The apparatus according to claim 15, wherein said image generation means (31) generates a two-dimensional image of the retina simultaneously with acquisition of the tomogram by said interferometer.

17. The apparatus according to claim 2, wherein curvatures, positions, and refractive indices of surfaces of lenses constituting a common portion between said first optical system (42, 43) and said second optical system (41, 42) so as to make at least a principal ray of the illumination light beam reflected by at least one lens surface of the lenses reach outside an imaging area of a light-receiving element of said light-receiving means.

18. The apparatus according to claim 17, wherein the lenses constituting the common portion between said first optical system (42, 43) and said second optical system (41, 42) are constituted by lens surfaces $S_1$, $S_2$..., $S_k$,..., $S_N$ (where $2 \leq k \leq N$: N is an integer not less than 2, and k increases toward a direction in which illumination light propagates toward an eye to be examined), and

when Lp represents a distance from light beam limiting means $S_A$ for limiting a return light beam from the eye to a position $S_P$ of a composite principal surface between the lens surfaces $S_1$,..., $S_{k-1}$,

$L_k$ represents a distance from the position Sp of the composite principal surface between the lens surfaces $S_1$,..., $S_{k-1}$ to the lens surface $S_k$,

$L_0$ represents a distance from $S_A$ to a principal surface $S_0$ of an imaging lens,

$f_0$ represents a focal length of the imaging lens; fp(k-1) represents a composite focal length between the lens surfaces $S_1$ to $S_{k-1}$,

$R_k$ represents a curvature radius of the lens surface $S_k$,

D represents an area used for detection by said light-receiving means,

W represents a beam radius of reflected light from the lens surface $S_k$ on a detection surface of said light-receiving means,

$y_{1A}$ represents a ray height of a principal ray of illumination light at a position of said light beam limiting means $S_A$, and

$U_{1P}$ represents an angle relative to an optical axis of an optical system,

the following relationship is satisfied:

```
yim > D/2 + W
```

for

```
yim = [-f0•[{(1 - 2•Lk/Rk)•(1-Lk/fP(k-1))

- Lk/fP(k-1)}/fP(k-1) + {2•(1-Lk

/fP(k-1))/Rk+1/fP(k-1)}]]•y1A

+ [-f0•[{-(1 - 2•Lk/Rk)•(1-Lk/fP(k-1)

+ Lk)•LP-Lk/fP(k-1)}/fP(k-1)

+ {-2•(1-Lk/fP(k-1) + Lk)•LP/Rk

+ (1-LP/fP(k-1))}]]•u1P
```

19. A method of controlling an ophthalmic apparatus comprising a first optical system (42, 43) and image generation means (31), the method comprising:

an irradiation step of causing the first optical system (42, 43) to irradiate the same region on a retina with a plurality of illumination light beams through separate positions on a pupil of an eye to be examined; and

an image generation step of causing the image generation means (31) to generate a two-dimensional image of the retina based on return light from the eye.

20. A non-transitory computer-readable storage medium storing a computer program for causing a computer to execute each step in a method of controlling an ophthalmic apparatus defined in claim 19.

# F I G. 1

EP 2 517 618 A2

# F I G. 2

# F I G. 3

# F I G. 4

# FIG. 5

EP 2 517 618 A2

# F I G. 6

# F I G. 7

# F I G. 8

FIG. 9

FIG. 10

FIG. 11

F I G. 12

F I G. 13

**EP 2 517 618 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 06046627 A
- US 6758564 B
- JP 2005531346 A